(19) Europäisches Patentamt
European Patent Office
Office européen des brevets

(11) EP 3 473 252 A1

(12) EUROPEAN PATENT APPLICATION
published in accordance with Art. 153(4) EPC

(43) Date of publication:
**24.04.2019 Bulletin 2019/17**

(21) Application number: **17814426.7**

(22) Date of filing: **15.02.2017**

(51) Int Cl.:
*A61K 31/506* (2006.01) *C07D 417/02* (2006.01)
*A61K 9/08* (2006.01) *A61K 9/10* (2006.01)
*A61K 9/107* (2006.01) *A61K 9/14* (2006.01)
*A61K 9/19* (2006.01) *A61K 9/20* (2006.01)
*A61K 9/48* (2006.01) *A61P 35/00* (2006.01)
*A61P 35/02* (2006.01) *A61P 19/08* (2006.01)

(86) International application number:
**PCT/CN2017/073645**

(87) International publication number:
**WO 2017/219687 (28.12.2017 Gazette 2017/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.06.2016 CN 201610450691**

(71) Applicant: **Shenzhen Haibin Pharmaceutical Co., Ltd.**
**Guangdong 518081 (CN)**

(72) Inventors:
- **CAI, Weihui**
  **Shanghai 201203 (CN)**
- **JIN, Fang**
  **Shanghai 201203 (CN)**

(74) Representative: **Schiweck Weinzierl Koch Patentanwälte Partnerschaft mbB**
**Ganghoferstraße 68 B**
**80339 München (DE)**

# (54) PYRIMIDINE COMPOUND OR SALT THEREOF IN APPLICATION FOR MANUFACTURING PHARMACEUTICAL PRODUCT FOR PREVENTING AND/OR TREATING FLT3-RELATED DISEASE OR DISORDER

(57) The present invention relates to a pyrimidine compound or salt thereof in an application for manufacturing a pharmaceutical product for preventing and /or treating an FLT3- related disease or disorder. The pyrimidine compound has a chemical structure as represented by formula (I).

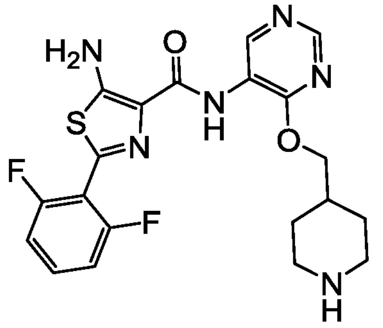

Formula (I)

Figure 3

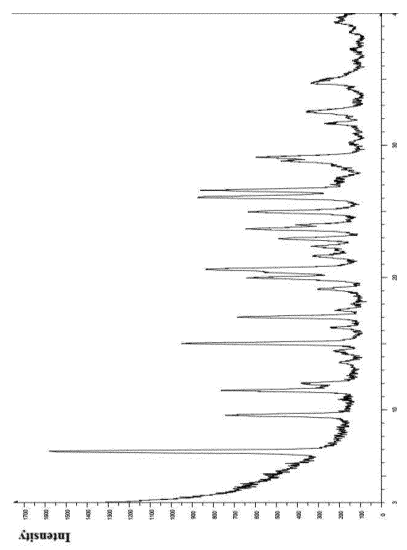


EP 3 473 252 A1

## Description

## CROSS-REFERENCE TO RELATED APPLICATIONS

The present application claims the priority and benefit of Chinese Patent Application No. 201610450691.4, filed on 21 June, 2016, entitled "PYRIMIDINE COMPOUND OR SALT THEREOF IN APPLICATION FOR MANUFACTURING PHARMACEUTICAL PRODUCT FOR PREVENTING AND /OR TREATING FLT3-RELATED DISEASE OR DISORDER", which is incorporated herein by reference in its entirety.

## TECHNICAL FIELD

The invention relates to a use of pyrimidine compounds or salts thereof in preparing drugs for the prevention and/or treatment of FLT3-related diseases or disorders.

## BACKGROUND ART

Malignant tumor is one of the most health-threatening diseases, according to *"2013Chinese Cancer Registry Annual Report"* recently released by the National Cancer Registry Center, the number of new cancer cases in China was as high as 3.09 million per year, the lifetime risk of cancer would be as high as 22% on the basis of average life expectancy of 74 years, thus cancer has become common disease. One of every five deaths in China died from cancer, accounting for one-fourth of all cancer deaths in the worldwide, cancer has become the second biggest killer after cardiovascular diseases. Although in recent years great progress has been made in cancer clinical treatment methods, such as chemotherapy, radiotherapy, molecule-targeted therapy and so on, the long-term survival rate of cancer has not been significantly improved, thus the development of new anti-cancer drugs is still a hot spot in pharmaceutical research and development.

Recently, protein kinase inhibitors have been a hot area in the research and development of anti-cancer drugs, many small-molecule kinase inhibitors, such as imatinib, sorafenib and sunitinib, are on the market, still a large number of small-molecule kinase inhibitors are in clinical research stage. FMS-like tyrosine kinase (FLT3), also called Fetal Liver Kinase-2 (FLK2) and Stem Cell Kinase-1 (STK1), is a Class III receptor tyrosine kinase. FLT3 is a receptor tyrosine kinase, whose nature ligand includes platelet derived growth factor (PDGF), colony stimulating factor 1 (CSF-1), and kit ligand (KL), when the ligand binds to FLT3, FLT3 is dimerized, at the same time intracellular tyrosine kinase domain is phosphorylated and accordingly activated, which activates downstream phosphatidyl inositol 3-kinase (PI3K) pathway and Ras pathway, promotes cell proliferation and division, and accelerates activation of BCL2 family protein BAD (BCL2 associated death promoter), an anti-apoptotic protein, consequently cell apoptosis is inhibited.

The interaction of FLT3 with many proteins including SH2-containing sequence proteins (SHCs), SH2-domain-containing inositol phosphatase (SHIP), SH2-domain-containing protein tyrosine phosphatase-2 (SHP2), Cbl and GRB2-associated binder (GAB2) can regulate activities of PI3K. Activated PI3K is capable of activating the downstream singles including 3-phosphoinositide-dependent kinase-1 (PDK1), protein kinase B (Akt1/PKB) and mammalian target of rapamycin (mTOR), further activating ribosome S6K protein kinase (S6K) and inhibiting eukaryotic cell initiation factor 4E binding protein 1 (4E-BP1), finally promoting transcription and translation of key genes. Meanwhile, FLT3 is able to mediate Ras pathway, activated FLT3, associating with growth factor receptor bound 2, activates Ras through SHC, then activates downstream signals including Raf, mitogen activated protein kinase (MAPK/ERK Kinases, MEK), p38, extracellular signal-regulated kinase 1/2 (ERK1/2) and ribosomal S6 Kinase (RSK), which can activate cAMP response element binding protein (CREB), Elk (also called erythropoietin-producing hepatocellular receptor B1 (EPH receptor B1)) and signal transducer and activator of transcription (STAT), eventually make proteins necessary to cell proliferation transcribed and translated, which promote proliferation of pluripotent stem cells, early progenitors and immature lymphocytes.

FLT3 relates to quite many diseases, such as tumor, cancer and hematological malignancies (Ansari-Lari, Ali. FLT3 mutations in myeloid sarcoma. British Journal of Haematology. 126(6):785-91). As a target of research and development of anti-cancer drugs, FLT3 has attracted a wide interest from academia, especially industry. Many FLT3 inhibitors with different structures have been identified, four of which have been approved for market, while still more candidate compounds are undergoing clinical trials. Now, FLT3 inhibitors on the market are mainly used for the treatment of various kinds of tumor. As an anti-cancer drug, FLT3 inhibitor is applied widely because of its significant efficacy and controllable side effects. Among the four FLT3 inhibitors on the market, the sales of sorafenib (marketed in 2005) and sunitini (marketed in 2006) in 2013 were $123 million and $124 million respectively, both exceeding $1 billion. The sales of ponatinib marketed in 2013 reached $45.2 million in its first year. Cabotini was marketed in 2013 and its sales reached $15 million in the first year. Therefore, as anti-cancer drug, FLT3 inhibitors have exhibited promising sales prospects.

## SUMMARY OF THE INVENTION

The applicant unexpectedly discovered that 5-amino-2-(2,6-difluorophenyl)-N-(4-(piperidine-4-methoxy)pyrimidine-5-) thiazole-4-formamide (compounds shown in Formula (I)) or pharmaceutically acceptable salts thereof had strong inhibiting activity against FLT3 kinase, therefore, the purposes of the present invention are to provide a use of the compound shown in Formula (I) or pharmaceutically acceptable salts thereof in preparing drugs for the prevention and/or treatment of FLT3-related diseases or disorders.

The technical solution of the present invention is described as below:

The present invention provides a use of a compound shown in Formula (I) or pharmaceutically acceptable salts thereof in preparing drugs for the prevention and/or treatment of FLT3-related diseases or disorders;

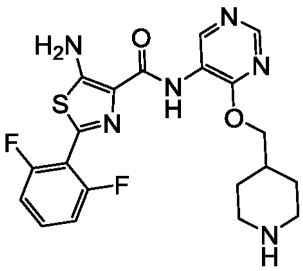

Formula (I)

wherein the compound shown in Formula (I) is 5-amino-2-(2,6-difluorophenyl)-N-(4-(piperidine-4-methoxy)pyrimidine-5-) thiazole-4-formamide, wherein the pharmaceutically acceptable salts are the common salts in the field of pharmacy, which may be salts formed from physiologically compatible organic and inorganic acids, these acids form nontoxic acid addition salts containing pharmaceutically acceptable anions, preferably, the pharmaceutically acceptable salts include but not limit to hydrochloride salt, hydrobromide, maleate, phosphate, succinate, sulphate, citrate, benzoate, mesylate, lactate, acetate, tosylate, palmitate, fumarate, tartrate, ascorbate, nitrate, formate, propionate, n-butyrate, isobutyrate, salicylate, oxalate, succinate, malate, glutamate, aspartate or gluconate;

more preferably, the pharmaceutically acceptable salts are hydrochloride salt, phosphate, mesylate, lactate, acetate, sulphate or hydrobromide;

most preferably, the pharmaceutically acceptable salt is hydrochloride salt. Preferably, the pharmaceutically acceptable salts are present in a crystalline form;

preferably, when the pharmaceutically acceptable salt is hydrochloride salt, the X-ray powder diffraction pattern of the crystalline form of the hydrochloride salt includes the diffraction peaks at $2\theta$ of 6.8±0.2°, 9.5±0.2°, 11.4±0.2°, 15.0±0.2°, 17.0±0.2°, 19.9±0.2°, 20.3±0.2°, 20.6±0.2°, 22.9±0.2°, 23.6±0.2°, 24.9±0.2°, 26.1±0.2°, 26.6±0.2°;

more preferably, the X-ray powder diffraction pattern of the crystalline form of the hydrochloride salt further includes the diffraction peaks at $2\theta$ of 12.0±0.2°, 28.8±0.2°, 29.1±0.2°, 32.5±0.2°, 34.7±0.2°.

Further preferably, the X-ray powder diffraction pattern of the crystalline form of the hydrochloride salt is shown as Figure 3.

Wherein the compound shown in Formula (I) or the pharmaceutically acceptable salts thereof can be administrated to a mammal (for example a human being) through oral administration, intravenous injection, intramuscular injection, subcutaneous injection or local administration as an active component.

Preferably, the drugs are solid preparations or liquid preparations, i.e. the compound shown in Formula (I) or the pharmaceutically acceptable salts thereof can be prepared into solid preparations or liquid preparation;

preferably, the solid preparations are selected from capsule, tablet, pill, powder, granule, sugar pill, and lyophilized preparation;

preferably, the liquid preparations are selected from solution, injection, suspension, oral liquid, syrup, tincture, and emulsion.

Wherein the FLT3-related diseases or disorders include FLT3 receptors related diseases, diseases involving FLT3 activity, or conditions associated with above-mentioned diseases.

Preferably, the FLT3-related diseases or disorders are tumors produced from abnormal or uncontrolled cell growth, including but not limited to hematopoietic dysfunction, specifically myelodysplastic disorders, such as thrombocytosis, essential thrombocytosis (ET), idiopathic extramedullary metaplasia, myelofibrosis (MF), myelofibrosis with myeloid metaplasia (MMM), chronic idiopathic myelofibrosis (IMF), polycythemia vera (PV), hematopenia and malignant

anterior spinal cord dysplasia syndrome. Preferably, the FLT3-related diseases or disorders are selected from glioma, lung cancer, breast cancer, colorectal cancer, prostate cancer, gastric cancer, esophageal cancer, colon cancer, pancreatic cancer, ovarian cancer, kidney cancer, thyroid cancer, neuron cancer and uterine cancer.

Preferably, the FLT3-related diseases or disorders are selected from leukemia, lymphoma and myeloma;

Preferably, the leukemia is selected from acute myeloid leukemia (AML), acute lymphoblastic leukemia (ALL), acute promyelocytic leukemia (APL), chronic lymphoblastic leukemia (CLL), chronic myeloid leukemia (CML), chronic neutrophilic leukemia (CNL), acute undifferentiated cell leukemia (AUL), prolymphocytic leukemia (PML), juvenile myelomonocytic leukemia (JMML), adult T-cell acute lymphocytic leukemia, acute myeloid leukemia with trilineage myelodysplasia (AML/TMDS), mixed lineage leukemia (MLL), and acute mononuclear leukemia (AMOL);
preferably, the lymphoma is non-Hodgkin lymphoma or Hodgkin lymphoma, for example degenerative large cell lymphoma;
preferably, the plasma cell diseases include multiple myeloma, macroglobulinemia or heavy chain disease;
preferably, the myeloma is selected from myelodysplastic syndrome (MDS), myelodysplastic disorder (MPD), multiple myeloma (MM) and spinal cord sarcoma.

In addition, the present invention also provides a method for preventing and/or treating FLT3-related diseases or disorders comprising administering a therapeutically effective amount of the compound shown in Formula (I) or the pharmaceutically acceptable salts thereof to a subject in need;
preferably, the subject is a mammal.

Preferably, the pharmaceutically acceptable salts are hydrochloride salt, hydrobromide, maleate, phosphate, succinate, sulphate, citrate, benzoate, mesylate, lactate, acetate, tosylate, palmitate, fumarate, tartrate, ascorbate, nitrate, formate, propionate, butyrate, isobutyrate, salicylate, oxalate, succinate, malate, glutamate, aspartate or gluconate;
preferably, the pharmaceutically acceptable salts are hydrochloride salt, phosphate, mesylate, lactate, acetate, sulphate or hydrobromide;
preferably, the pharmaceutically acceptable salt is hydrochloride salt; preferably, the pharmaceutically acceptable salts are present in a crystalline form;
preferably, when the pharmaceutically acceptable salt is hydrochloride salt, the X-ray powder diffraction pattern of the crystalline form of the hydrochloride salt includes the diffraction peaks at 2θ of 6.8±0.2°, 9.5±0.2°, 11.4±0.2°, 15.0±0.2°, 17.0±0.2°, 19.9±0.2°, 20.3±0.2°, 20.6±0.2°, 22.9±0.2°, 23.6±0.2°, 24.9±0.2°, 26.1±0.2°, 26.6±0.2°;
more preferably, the X-ray powder diffraction pattern of the crystalline form of the hydrochloride salt further includes the diffraction peaks at 2θ of 12.0±0.2°, 28.8±0.2°, 29.1±0.2°, 32.5±0.2°, 34.7±0.2°;
Further preferably, the X-ray powder diffraction pattern of the crystalline form of the hydrochloride salt is shown as Figure 3.

Preferably, the FLT3-related diseases or disorders are myelodysplastic disorders, such as thrombocytosis, essential thrombocytosis, idiopathic extramedullary metaplasia, myelofibrosis, myelofibrosis with myeloid metaplasia, chronic idiopathic myelofibrosis, polycythemia vera, hematopenia and malignant anterior spinal cord dysplasia syndrome;
preferably, the FLT3-related diseases or disorders are selected from glioma, lung cancer, breast cancer, colorectal cancer, prostate cancer, gastric cancer, esophageal cancer, colon cancer, pancreatic cancer, ovarian cancer, kidney cancer, thyroid cancer, neuron cancer and uterine cancer;
preferably, the FLT3-related diseases or disorders are selected from leukemia, lymphoma and myeloma;
preferably, the leukemia is selected from acute myeloid leukemia, acute lymphoblastic leukemia, acute promyelocytic leukemia, chronic lymphoblastic leukemia, chronic myeloid leukemia, chronic neutrophilic leukemia, acute undifferentiated cell leukemia, prolymphocytic leukemia, juvenile myelomonocytic leukemia, adult T-cell acute lymphocytic leukemia, acute myeloid leukemia with trilineage myelodysplasia, mixed lineage leukemia, and acute mononuclear leukemia;
preferably, the lymphoma is non-Hodgkin lymphoma or Hodgkin lymphoma, for example degenerative large cell lymphoma;
preferably, the plasma cell diseases include multiple myeloma, macroglobulinemia or heavy chain disease;
preferably, the myeloma is selected from myelodysplastic syndrome, myelodysplastic disorder, multiple myeloma and spinal cord sarcoma.

## BRIEF DESCRIPTION OF THE DRAWINGS

Hereinafter, the embodiments of the present invention will be described in detail with reference to the accompanying drawings, wherein:

Figure 1 shows the $^1$HNMR spectrum of the hydrochloride salt of the compound shown in formula (I) prepared in Example 2;
Figure 2 shows the mass spectrum of the hydrochloride salt of the compound shown in formula (I) prepared in Example 2; and
Figure 3 shows the X-ray powder diffraction pattern of the crystalline form of the hydrochloride salt of the compound

shown in formula (I) prepared in Example 2.

## DETAILED DESCRIPTION OF THE EMBODIMENTS

Hereinafter, the present invention will be further illustrated in conjunction with the specific examples. It should be understood that the examples of the present invention are only used for explaining the present invention, rather than limiting the scope of the present invention. The experimental methods without specific conditions in the following examples are usually carried out according to the conventional conditions or the conditions of suggested by manufacturers.

Unless otherwise defined, all professional and scientific terms in the description have the same meaning that is familiar to those skilled in the art. In addition, any methods and materials similar or equivalent to those described herein can be used in the method of the present invention. The preferred implementation method and materials herein are only used for demonstration.

## EXAMPLE 1 Preparation of the compound shown in Formula (I)

1) Preparation of tert-butyl 4-((5-amino-pyrimidinyl-4-oxy) methyl) piperidine-1-carboxylate (SM-3)

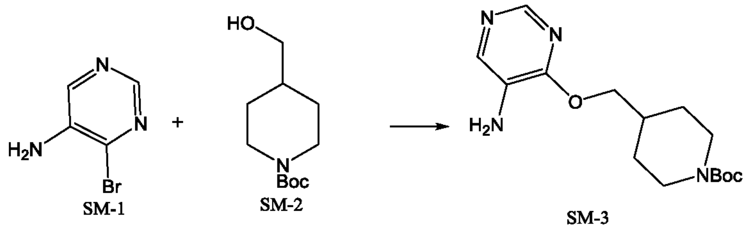

SM-1 SM-2 SM-3

At room temperature (2°C), NaH (71mg, 2.94mmol) was added to tert-butyl 4-hydroxymethyl-piperidine-1-carboxylate (SM-2) (574mg, 2.67mmol) in THF (tetrahydrofuran) (10mL) and stirred for 1 hour, and then 4-bromopyrimidine-5-amine (SM-1) (348mg, 2.67mmol) was added thereinto. The reactant was heated to 100°C under nitrogen protection, stirred for 4 hours, and then concentrated in a vacuum rotatory evaporator at room temperature (20-30 °C). The residue after concentration was purified by silica gel chromatography (the used eluent was: 10-30% ethyl acetate/petrol) to give tert-butyl 4-((5-amino-pyrimidinyl-4-oxy) methyl) piperidine-1-carboxylate (SM-3) (370mg, 1.2mmol).

2) Preparation of tert-butyl 4-((5-(5-amino-2-(2, 6-difluorophenyl) thiazole-4-carboxamide) pyrimidine-4-oxy) methyl) piperidine-1-carboxylate (SM-5)

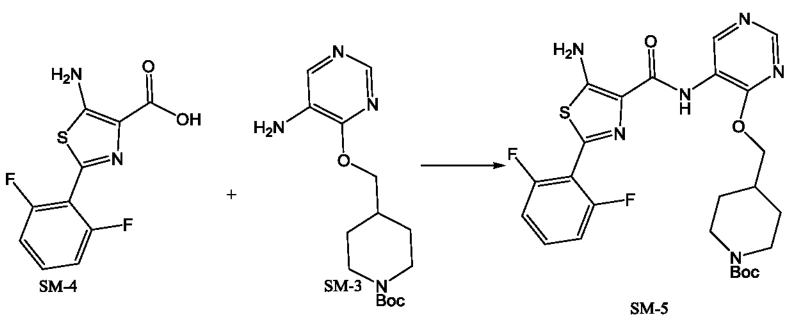

SM-4 SM-3 SM-5

A mixture of compound (SM-3) (52mg, 0.169mmol), compound 5-amino-2-(2,6-difluorophenyl) thiazole-4-carboxylic acid (1E) (SM-4) (40mg, 0.169mmol), HATU (77mg, 0.203mmol) and DIEA (93 μL, 0.507mmol) in DMF (5mL) was stirred for 1 hour at 50°C, diluted with ethyl acetate (50mL) after cooling and then washed with saturated salt water. The organic phase was concentrated in a vacuum rotatory evaporator at room temperature (20-30 °C) after drying with $Na_2SO_4$. The residue after concentration was purified by silica gel chromatography (the used eluent was: 10-30% ethyl acetate/petrol) to give tert-butyl 4-((5-(5-amino-2- (2, 6-difluorophenyl) thiazole-4-carboxamide) pyrimidine-4- oxy) methyl) piperidine-1-carboxylate (SM-5) (32mg, 0.0585mmol).

3) Preparation of 5-amino-2-(2, 6-difluorophenyl)-N-(4-(piperidine-4-methoxy) pyrimidine-5-) thiazole-4-formamide (Formula I)

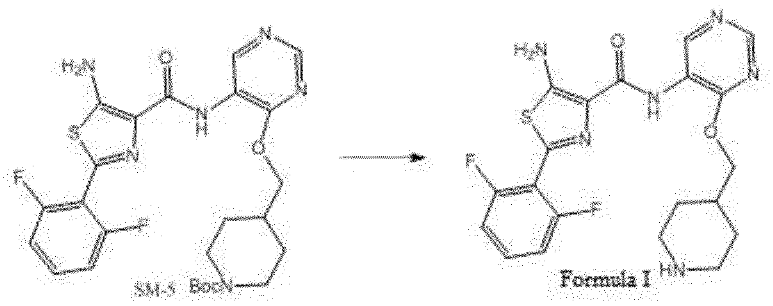

At room temperature (25°C), TFA (trifluoracetic acid) (0.5mL) was added to compound (SM-5) (21mg, 0.0384mmol) in $CH_2Cl_2$ (1mL), stirred for 10 minutes, and then concentrated in a vacuum rotatory evaporator at room temperature (25 °C), the residue was dissolved in $CH_2Cl_2$ (10mL), washed respectively with 1 equivalent NaOH (5mL) and saturated salt water (5mL), and the organic phase was dried with $Na_2SO_4$, and then concentrated in a vacuum rotatory evaporator at room temperature (25 °C) to give the product of 5-amino-2-(2, 6-difluorophenyl)-N-(4-(piperidine-4-methoxy) pyrimidine-5-) thiazole-4-formamide (the compound shown in formula (I)) (11mg, 0.0246mmol).
1H NMR (400MHz, CD3OD): δ ppm 1.26-1.29 (m, 2H), 1.91-1.94 (m, 2H), 2.04-2.12 (m, 1H), 2.66-2.69 (m, 2H), 3.10-3.13 (m, 2H), 3.90-3.98 (m, 2H), 6.99-7.02 (m, 2H), 7.31-7.41 (m, 1H), 8.33 (s, 1H), 9.40 (s, 1H).
MS (ESI) 447m/z $(M+H)^+$.

**EXAMPLE 2 Synthesis of hydrochloride salt of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, 165μl of IN methanol solution of hydrochloric acid(0.165mmol) was added in one portion, the solution became muddy slowly, stirred for half an hour at 20~30°C, filtered,and then the filter cake was dried under vacuum at 50°C to obtain 30mg of almost white solid with a yield of 56.5%, mp: 236.1~239.4°C.

The $^1$HNMR (400MHz, DMSO-$d_6$) spectrum of the obtained almost white solid was shown as Fig. 1, the mass spectrum was shown as Fig. 2, wherein m/z: 446.9 [(M-HCl)+H]+.

The obtained almost white solid is a hydrochloride salt of the compound shown in Formula (I), which is in a crystalline form, and the X-ray powder diffraction pattern of the crystal was shown as Fig. 3, wherein the detecting condition is shown as below and the detecting results are listed in Table 1:

Detecting apparatus: Bruker D8AdvanceX-ray diffractometer
Detecting conditions: target material was Cu, 2θ scan started at 3.000, 2θ scan ended at 40.000, the voltage was 40KV, the current was 40mA, Ka1=1.54060, Ka2 =1.54439, Ka2/Ka1 = 0.5, Ka = 1.54186.

Table 1 X-ray Diffraction Pattern Datum of the Crystalline Form of Hydrochloride Salt of Compound Shown in Formula (I)

| No. | Angle 2θ | Counts | Intensity (%) |
|---|---|---|---|
| 1 | 6.8 | 1590 | 100 |
| 2 | 9.5 | 747 | 47 |
| 3 | 11.4 | 760 | 47.8 |
| 4 | 12.0 | 391 | 24.6 |
| 5 | 15.0 | 957 | 60.2 |
| 6 | 17.0 | 692 | 43.5 |
| 7 | 19.9 | 659 | 41.4 |

(continued)

| No. | Angle 2θ | Counts | Intensity (%) |
|---|---|---|---|
| 8 | 20.3 | 547 | 34.4 |
| 9 | 20.6 | 846 | 53.2 |
| 10 | 22.9 | 491 | 30.9 |
| 11 | 23.6 | 642 | 40.4 |
| 12 | 24.9 | 635 | 39.9 |
| 13 | 26.1 | 870 | 54.7 |
| 14 | 26.6 | 861 | 54.2 |
| 15 | 28.8 | 475 | 29.9 |
| 16 | 29.1 | 597 | 37.5 |
| 17 | 32.5 | 365 | 23 |
| 18 | 34.7 | 345 | 21.7 |

**EXAMPLE3 Synthesis of phosphate of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30 °C, a mixed solution of 5mg phosphoric acid (0.051mmol) and 0.5ml methanol was added in one portion, the solution became muddy slowly, stirred for half an hour at 20~30°C, filtered, and then the filter cake was dried under vacuum at 50°C to obtain 50mg of almost white solid with a yield of 94.9%, mp: 222.3~224.8°C, m/z: 446.9[(M-$H_3PO_4$)+H]+.

**EXAMPLE 4 Synthesis of mesylate of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, a mixed solution of 16mg methanesulfonic acid (0.166mmol) and 0.5ml methanol was added in one portion, the solution became muddy slowly, stirred for half an hour at 20~30°C, the solvent was evaporated under reduced pressure at 50 °C, 10ml dichloromethane was added and stirred for half an hour at 20~30°C, filtered, and then the filter cake was dried under vacuum at 50°C to obtain 37mg of almost white solid with a yield of 62%, mp: 242.1~246.7 °C, m/z: 446.9[(M-$CH_4O_3S$)+H]+.

**EXAMPLE 5 Synthesis of lactate of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, a mixed solution of 15mg lactic acid (0.167mmol) and 0.5ml methanol was added in one portion, the solution was clear, stirred for half an hour at 20~30°C, the solvent was evaporated under reduced pressure at 50°C, 10ml dichloromethane was added and stirred for half an hour at 20~30°C, filtered, and then the filter cake was dried under vacuum at 50°C to obtain 36mg of almost white solid with a yield of 61%, mp: 210.1~213.6°C, m/z: 446.9[(M-$C_3H_6O_3$)+H]+.

**EXAMPLE 6 Synthesis of acetate of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, a mixed solution of 10mg acetic acid (0.167mmol) and 0.5ml methanol was added in one portion, the solution was clear, stirred for half an hour at 20~30°C, the solvent was evaporated under reduced pressure at 50°C, 10ml dichloromethane was added and stirred for half an hour at 20~30°C, filtered, and then the filter cake was dried under vacuum at 50°C to obtain 31mg of almost white solid with a yield of 55.6%, mp: 232.2~233.7°C, m/z: 446.9 [(M-$CH_3COOH$)+H]+.

**EXAMPLE 7 Synthesis of sulphate of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, a mixed solution of 8mg sulphuric acid (0.081mmol) and 0.5ml methanol was added in one portion, the solution became muddy slowly, stirred for half an hour at 20~30°C, filtered, and then the filter cake was dried under vacuum at 50°C to obtain 53mg of almost white solid with a yield of 88.5%, mp: 231.5~236.9 °C , m/z: 446.9[(M-$H_2SO_4$)+H]+.

**EXAMPLE 8 Synthesis of hydrobromide of the compound shown in Formula (I)**

50mg of the compound shown in Formula (I) prepared in EXAMPLE 1(0.11mmol), 2ml methanol and 8ml dichloromethane were added into a reaction bottle, stirred until the solution became clear at 20~30°C, a mixed solution of 30% 44mg hydrobromic acid (0.163mmol) and 0.5ml methanol was added in one portion, the solution became muddy slowly, stirred for half an hour at 20~30°C, filtered, then the filter cake was dried under vacuum at 50°C to obtain 24mg of almost white solid with a yield of 41.3%, mp:225.1~227.7 °C, m/z: 446.9[(M-HBr)+H]+.

**EXAMPLE 9 Activity of inhibiting FLT3 Kinase**

The used abbreviations in the following experiments represent the following meanings:

HEPES: 4-(2-hydroxyethyl)-1-piperazineethanesulfonic acid;
Brij-35: polyethylene glycol dodecyl ether;
DTT: dithiothreitol;
EDTA: ethylene diamine tetraacetic acid;
EGFR: epidermal growth factor receptor;
HER2: human epidermal growth factor receptor 2;
EGFRT790M: epidermal growth factor receptor T790M mutant;
Peptide FAM-P22: fluorescein labeled peptide 22;
ATP: adenosine triphosphate;
DMSO: dimethyl sulfoxide;
Staurosporine: staurosporine;
Coating Reagent#3: coating reagent#3.

1. Preparation of 1×Kinase Buffer and Termination Buffer:

(1) $MnCl_2$-free 1×kinase buffer: 50mM HEPES, pH 7.5, 0.0015% Brij-35, 10mM $MgCl_2$, 2mM DTT;
(2) Termination buffer: 100mM HEPES, pH 7.5, 0.015% Brij-35, 0.2% Coating Reagent#3, 50mM EDTA.

2. Preparation of compounds for testing kinase: the compounds are diluted continuously.

(1) the compound was diluted to 50 times the maximum final concentration with 100% DMSO. 100 mL of the solution containing the compound at this concentration was transferred to one hole of 96-well plate;
(2) 10 concentrations of compounds were prepared by diluting 20 mL original solution with 60 mL DMSO;
(3) 100mL solution of 100% DMSO was added into two empty holes as compound-free control and enzyme-free control;
(4) a transitional plate was prepared, 10mL compounds at different concentrations were transferred from the original plate to the transitional plate respectively, 90mL 1×kinase buffer was added, and then mixed under oscillation for 10 minutes;
(5) preparation of an experimental plate: 5mL solution containing compounds was transferred from the hole of the transitional plate of the 96-well plate to the corresponding hole of 384-well plate.

3. Kinase reaction

(1)Preparation of 2.5×enzyme solution: enzyme was added to the 1×kinase buffer;
(2)Preparation of 2.5×peptide solution: a fluorescein labeled peptide and ATP were added to the 1×kinase buffer;
(3)10mL 2.5×enzyme solution was added to an experimental 384-well plate containing the compound solution with a concentration of 10% containing 5mL DMSO and incubated at room temperature for 10 minutes;
(4)10mL 2.5×peptide solution was added to an experimental 384-well plate;
(5) kinase reaction and termination: Incubation was carried out at 28°C for certain time, 25mL termination buffer was added to terminate the reaction.

4. Detection of data

Data was read and collected.

5. Fitting of curve

(1) detected data was copied and converted
(2) data was converted to inhibitory rate

$$\text{Inhibitory rate}=(\text{Maximum Value}-\text{Sample Value})/(\text{Maximum Value}-\text{Minimum Value})*100;$$

wherein "Maximum Value" is the value of DMSO control; "Minimum Value" is the value of kinase-free control hole.
(3) the data was input an analysis software to obtain $IC_{50}$ value. The experimental results were shown as Table 2:

Table 2 Inhibiting Activity of Compounds of the Present Invention Against FLT3 Kinase

| Samples | FLT3($IC_{50}$,nM) |
|---|---|
| Compound of Formula (I) (prepared in EXAMPLE 1) | 40 |
| Hydrochloride salt of compound of Formula (I) (prepared in EXAMPLE 2) | 15 |
| Phosphate of compound of Formula (I) (prepared in EXAMPLE 3) | 17 |
| Mesylate of compound of Formula (I) (prepared in EXAMPLE 4) | 28 |
| Lactate of compound of Formula (I) (prepared in EXAMPLE 5) | 25 |
| Acetate of compound of Formula (I) (prepared in EXAMPLE 6) | 27 |
| Sulphate of compound of Formula (I) (prepared in EXAMPLE 7) | 28 |
| Hydrobromide of compound of Formula (I) (prepared in EXAMPLE 8) | 39 |

The results show that the compounds of the present invention had quite strong activities of inhibiting FLT3 in vitro.

**EXAMPLE 10 Effects on proliferation of tumor cells**

This example tested the effects of the compounds of the present invention (the compound shown in Formula (I), hydrochloride salt, phosphate, mesylate, lactate, acetate, sulphate, and hydrobromide thereof prepared in EXAMPLES 1-8 respectively) on proliferation of tumor cells, and further evaluated the inhibitory effects of the compounds on proliferation of tumor cells and selectivity of the compounds in inhibiting proliferation of tumor cells.
The present invention selected the following cells:

(1) human acute monocytic leukemia cell strain MV4-11 (expressing FLT3/ITD mutant gene);
(2) human acute myeloid leukemia cell strain MOLM-13 (expressing FLT3/ITD mutant gene and wild-type FLT3 gene);
(3) human acute myeloid leukemia cell strain MOLM-14 (expressing FLT3/ITD mutant gene and wild-type FLT3 gene);
(4) human acute myeloid leukemia cell strain OCI-AML-3 (expressing FLT3A680V mutant gene);
(5) human acute myeloid leukemia cell strain U937 (expressing wild-type FLT3 gene);
(6) mouse TEL-BaF3-FLT3/ITD (stably expressing FLT3/ITD mutant activated kinase);

(7) mouse TEL-BaF3-FLT3-D835Y (stably expressing FLT3D835Y mutant activated kinase);
(8) mouse TEL-FLT3-BaF3 (stably expressing FLT3 kinase);
(9) mouse BaF3-FLT3-ITD-D835Y (stably expressing FLT3/ITD D835Y mutant activated kinase);
(10) mouse BaF3-FLT3-ITD-F691L (stably expressing FLT3/ITD F691L mutant activated kinase).

The compounds at different concentration (0.000508μM, 0.00152μM, 0.00457μM, 0.0137μM, 0.0411μM, 0.123μM, 0.370μM, 1.11μM, 3.33μM, 10μM in DMSO) were added to the above-mentioned cells respectively, incubated for 72 hours, Cell Titer-Glo (Promega, United States of America) Chemiluminescence Kit was used to quantify ATP in living cells to detect the number of living cells, the results were shown as Table 3

Table 3 Effects of Compounds on Proliferation of Cells

| Cell | IC50(μM) | | | | | | | |
|---|---|---|---|---|---|---|---|---|
| | Compound of Formula (I) | Hydrochloride salt of compound of Formula (I) | Phosphate of compound of Formula (I) | Mesylate of compound of Formula (I) | Lactate of compound of Formula (I) | Acetate of compound of Formula (I) | Sulphate of compound of Formula (I) | Hydrobromide of compound of Formula (I) |
| MV4-11 | 0.51 | 0.13 | 0.33 | 0.43 | 0.34 | 0.41 | 0.23 | 0.42 |
| MOLM-13 | 0.52 | 0.12 | 0.31 | 0.45 | 0.28 | 0.43 | 0.23 | 0.51 |
| MOLM-14 | 0.27 | 0.08 | 0.12 | 0.13 | 0.31 | 0.12 | 0.22 | 0.13 |
| OCI-AML-3 | 0.34 | 0.05 | 0.12 | 0.06 | 0.08 | 0.32 | 0.21 | 0.11 |
| U937 | 13.41 | 6.21 | 6.43 | 7.42 | 9.05 | 2.09 | 3.56 | 4.66 |
| TEL, BaF3-FT3/ITD | 0.11 | 0.02 | 0.03 | 0.02 | 0.04 | 0.02 | 0.04 | 0.02 |
| FEL-BaF3-FLT3-D835Y | 0.32 | 0.21 | 0.22 | 0.31 | 0.13 | 0.32 | 0.22 | 0.31 |
| FEL-FLT3-BaF3 | 0.12 | 0.03 | 0.02 | 0.05 | 0.06 | 0.03 | 0.05 | 0.16 |
| 3aF3-FLT3-TD-D835Y | 0.61 | 0.32 | 0.23 | 0.55 | 0.34 | 0.53 | 0.23 | 0.66 |
| 3aF3-FLT3-TD-F691L | 0.93 | 0.45 | 0.76 | 0.77 | 0.64 | 0.94 | 0.56 | 0.76 |

## EXAMPLE 11 Therapeutic effects on subcutaneous xenograft tumor of human MV-4-11 in nude mice

Preparation of test samples: all samples were prepared with 5% anhydrous ethanol, 5% Cremophor EL and 90 % physiological saline.

Animals: BALB/cA-nude mice, 6-7 weeks, female ♀, purchased from Shanghai Lingchang Biotechnology Co., Ltd.. Production license number was SC×K(Hu)2013-0018; animal certification number was 2013001810589. Feeding environment: SPF Level.

Experiment steps: human acute myeloid leukemia cells MV-4-11 were inoculated subcutaneously in nude mice, after the tumor grows to 80-150mm$^3$, animals were randomly divided into groups with 6 mice each group. The dosage was 15mg/kg, the drugs were administrated by intraperitoneal injection, 3 times a day for 21 days. The volume of tumor was measured, the weight of mouse was weighed and the data were recorded.

The formula for calculating the volume of tumor (V) was:

$$V = 1/2 \times a \times b^2$$

Wherein a and b represented length and width respectively.

$$T/C\ (\%) = (T\text{-}T_0)\ /\ (C\text{-}C_0) \times 100$$

Wherein T and C were the volume of tumor when the experiment was completed; $T_0$ and $C_0$ were the volume of tumor when the experiment was started.

The inhibited rate of tumor was calculated, the results were shown as Table 4.

Table 4 Therapeutic Effects on MV-4-11 Cell of Subcutaneous Xenograft Tumor in Nude Mice

| Samples | Inhibited Rate of Tumor (%) |
|---|---|
| Compound of Formula (I) (prepared in EXAMPLE 1) | 66 |
| Hydrochloride salt of compound of Formula (I) (prepared in EXAMPLE 2) | 86 |
| Phosphate of compound of Formula (I) (prepared in EXAMPLE 3) | 69 |
| Mesylate of compound of Formula (I) (prepared in EXAMPLE 4) | 74 |
| Lactate of compound of Formula (I) (prepared in EXAMPLE 5) | 71 |
| Acetate of compound of Formula (I) (prepared in EXAMPLE 6) | 77 |
| Sulphate of compound of Formula (I) (prepared in EXAMPLE 7) | 68 |
| Hydrobromide of compound of Formula (I) (prepared in EXAMPLE 8) | 66 |

## EXAMPLE 12 Preparation of tablet

10g of hydrochloride salt of the compound of Formula (I) prepared in EXAMPLE 2, 40g of microcrystalline cellulose, 100g of lactose and 1g of polyvinylpolypyrrolidone were collected and screened through 80 mesh respectively, mixed evenly, 2% HPMC solution was used as a binder, screened through 18 mesh, granulated, dried, screened through 20 mesh and then broken, 1g of polyvinylpolypyrrolidone, 1g of micro powder silica gel and 1g of magnesium stearate were added, mixed for 10min, tableted (0.16g/tablet) to obtain a tablet.

## EXAMPLE 13 Preparation of capsule

10g of the compound of Formula (I) prepared in EXAMPLE 1, 40g of microcrystalline cellulose, 100g of lactose were collected and screened through 80 mesh respectively, mixed evenly, 1g of sodium carboxyl methyl starch and 1g of magnesium stearate were added, mixed for 10min, and then filled into a $3^{\#}$ capsule to obtain a capsule.

## EXAMPLE 14 Preparation of injection

30g of acetate of the compound of Formula (I) prepared in EXAMPLE 6 and 50g of glucose were added into

900ml of water for injection, stirred to dissolve, water for injection was added to 1000ml, stirred evenly, filtered through 0.22μm filter membrane, and then filled to ampoules at 1 ml/branch, sealed, sterilized under 121°C for 20 minutes to obtain an injection.

**EXAMPLE 15 Preparation of lyophilized preparation**

20g of hydrochloride salt of the compound of Formula (I) prepared in EXAMPLE 2 and 50g of mannitol were added into 900ml of water for injection, stirred to dissolve, water for injection was added to 1000ml, stirred evenly, filtered through 0.22μm filter membrane, and then filled to vials at 1ml/branch, freeze-dried, sealed to obtain a lyophilized preparation.

**EXAMPLE 16 Preparation of oral liquid**

20g of mesylate of the compound of Formula (I) prepared in EXAMPLE 4, 200g of sucrose and 1g of flavor were added into 900ml of water for injection, stirred to dissolve, water for injection was filled to 1000ml, stirred evenly, filtered through 0.22μm filter membrane, and then filled to oral liquid bottle at 1ml/branch to obtain an oral liquid preparation.

## Claims

1. Use of a compound shown in Formula (I) or pharmaceutically acceptable salts thereof in preparing drugs for the prevention and/or treatment of FLT3-related diseases or disorders:

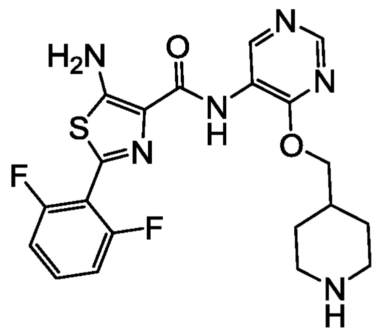

Formula (I).

2. The use according to claim 1, wherein the pharmaceutically acceptable salts are hydrochloride salt, hydrobromide, maleate, phosphate, succinate, sulphate, citrate, benzoate, mesylate, lactate, acetate, tosylate, palmitate, fumarate, tartrate, ascorbate, nitrate, formate, propionate, n-butyrate, isobutyrate, salicylate, oxalate, succinate, malate, glutamate, aspartate or gluconate;
preferably, the pharmaceutically acceptable salts are hydrochloride salt, phosphate, mesylate, lactate, acetate, sulphate or hydrobromide;
more preferably, the pharmaceutically acceptable salt is hydrochloride salt.

3. The use according to claim 1 or 2, wherein the pharmaceutically acceptable salts are present in a crystalline form;
preferably, when the pharmaceutically acceptable salt is hydrochloride salt, the X-ray powder diffraction pattern of the crystalline form of the hydrochloride salt includes the diffraction peaks at 2θ of 6.8±0.2°, 9.5±0.2°, 11.4±0.2°, 15.0±0.2°, 17.0±0.2°, 19.9±0.2°, 20.3±0.2°, 20.6±0.2°, 22.9±0.2°, 23.6±0.2°, 24.9±0.2°, 26.1±0.2°, 26.6±0.2°;
more preferably, the X-ray powder diffraction pattern of the crystalline form of the hydrochloride salt further includes the diffraction peaks at 2θ of 12.0±0.2°, 28.8±0.2°, 29.1±0.2°, 32.5±0.2°, 34.7±0.2°;
further preferably, the X-ray powder diffraction pattern of the crystalline form of the hydrochloride salt is shown as Figure 3.

4. The use according to any one of claims 1 to 3, wherein the drugs are solid preparations or liquid preparations;
preferably, the solid preparations are selected from capsule, tablet, pill, powder, granule, sugar pill, and lyophilized preparation;

preferably, the liquid preparations are selected from solution, injection, suspension, oral liquid, syrup, tincture, and emulsion.

**5.** The use according to any one of claims 1 to 4, wherein the FLT3-related diseases or disorders are myelodysplastic disorders, such as thrombocytosis, essential thrombocytosis, idiopathic extramedullary metaplasia, myelofibrosis, myelofibrosis with myeloid metaplasia, chronic idiopathic myelofibrosis, polycythemia vera, hematopenia and malignant anterior spinal cord dysplasia syndrome.

**6.** The use according to any one of claims 1 to 5, wherein the FLT3-related diseases or disorders are selected from glioma, lung cancer, breast cancer, colorectal cancer, prostate cancer, gastric cancer, esophageal cancer, colon cancer, pancreatic cancer, ovarian cancer, kidney cancer, thyroid cancer, neuron cancer and uterine cancer.

**7.** The use according to any one of claims 1 to 6, wherein the FLT3 -related diseases or disorders are selected from leukemia, lymphoma and myeloma; preferably, the leukemia is selected from acute myeloid leukemia, acute lymphoblastic leukemia, acute promyelocytic leukemia, chronic lymphoblastic leukemia, chronic myeloid leukemia, chronic neutrophilic leukemia, acute undifferentiated cell leukemia, prolymphocytic leukemia, juvenile myelomonocytic leukemia, adult T-cell acute lymphocytic leukemia, acute myeloid leukemia with trilineage myelodysplasia, mixed lineage leukemia, and acute mononuclear leukemia;
preferably, the lymphoma is non-Hodgkin lymphoma or Hodgkin lymphoma, for example degenerative large cell lymphoma;
preferably, the plasma cell diseases include multiple myeloma, macroglobulinemia or heavy chain disease;
preferably, the myeloma is selected from myelodysplastic syndrome, myelodysplastic disorder, multiple myeloma and spinal cord sarcoma.

**8.** A method for preventing and/or treating FLT3-related diseases or disorders comprising administering a therapeutically effective amount of the compound shown in Formula (I) or the pharmaceutically acceptable salts thereof to a subject in need;
preferably, the subject is a mammal.

**9.** The method according to claim 9, wherein, the pharmaceutically acceptable salts are hydrochloride salt, hydrobromide, maleate, phosphate, succinate, sulphate, citrate, benzoate, mesylate, lactate, acetate, tosylate, palmitate, fumarate, tartrate, ascorbate, nitrate, formate, propionate, butyrate, isobutyrate, salicylate, oxalate, succinate, malate, glutamate, aspartate or gluconate;
preferably, the pharmaceutically acceptable salts are hydrochloride salt, phosphate, mesylate, lactate, acetate, sulphate or hydrobromide;
preferably, the pharmaceutically acceptable salt is hydrochloride salt; preferably, the pharmaceutically acceptable salts are present in a crystalline form;
preferably, when the pharmaceutically acceptable salt is hydrochloride salt, the X-ray powder diffraction pattern of the crystalline form of the hydrochloride salt includes the diffraction peaks at $2\theta$ of $6.8\pm0.2°$, $9.5\pm0.2°$, $11.4\pm0.2°$, $15.0\pm0.2°$, $17.0\pm0.2°$, $19.9\pm0.2°$, $20.3\pm0.2°$, $20.6\pm0.2°$, $22.9\pm0.2°$, $23.6\pm0.2°$, $24.9\pm0.2°$, $26.1\pm0.2°$, $26.6\pm0.2°$;
more preferably, the X-ray powder diffraction pattern of the crystalline form of the hydrochloride salt further includes the diffraction peaks at $2\theta$ of $12.0\pm0.2°$, $28.8\pm0.2°$, $29.1\pm0.2°$, $32.5\pm0.2°$, $34.7\pm0.2°$;
Further preferably, the X-ray powder diffraction pattern of the crystalline form of the hydrochloride salt is shown as Figure 3.

**10.** The method according to claim 8 or 9, wherein the FLT3-related diseases or disorders are myelodysplastic disorders, such as thrombocytosis, essential thrombocytosis, idiopathic extramedullary metaplasia, myelofibrosis, myelofibrosis with myeloid metaplasia, chronic idiopathic myelofibrosis, polycythemia vera, hematopenia and malignant anterior spinal cord dysplasia syndrome;
preferably, the FLT3-related diseases or disorders are selected from glioma, lung cancer, breast cancer, colorectal cancer, prostate cancer, gastric cancer, esophageal cancer, colon cancer, pancreatic cancer, ovarian cancer, kidney cancer, thyroid cancer, neuron cancer and uterine cancer;
preferably, the FLT3-related diseases or disorders are selected from leukemia, lymphoma and myeloma;
preferably, the leukemia is selected from acute myeloid leukemia, acute lymphoblastic leukemia, acute promyelocytic leukemia, chronic lymphoblastic leukemia, chronic myeloid leukemia, chronic neutrophilic leukemia, acute undifferentiated cell leukemia, prolymphocytic leukemia, juvenile myelomonocytic leukemia, adult T-cell acute lymphocytic leukemia, acute myeloid leukemia with trilineage myelodysplasia, mixed lineage leukemia, and acute mononuclear leukemia;

preferably, the lymphoma is non-Hodgkin lymphoma or Hodgkin lymphoma, for example degenerative large cell lymphoma;
preferably, the plasma cell diseases comprise multiple myeloma, macroglobulinemia or heavy chain disease;
preferably, the myeloma is selected from myelodysplastic syndrome, myelodysplastic disorder, multiple myeloma and spinal cord sarcoma.

Figure 1

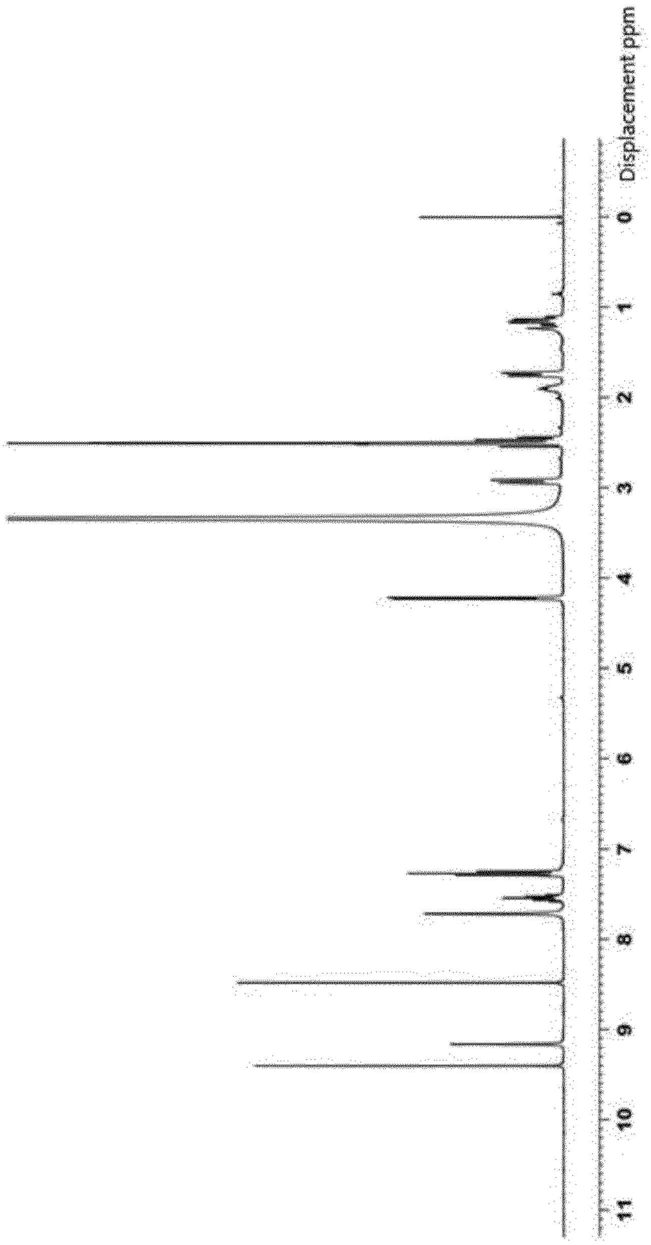

# Figure 2

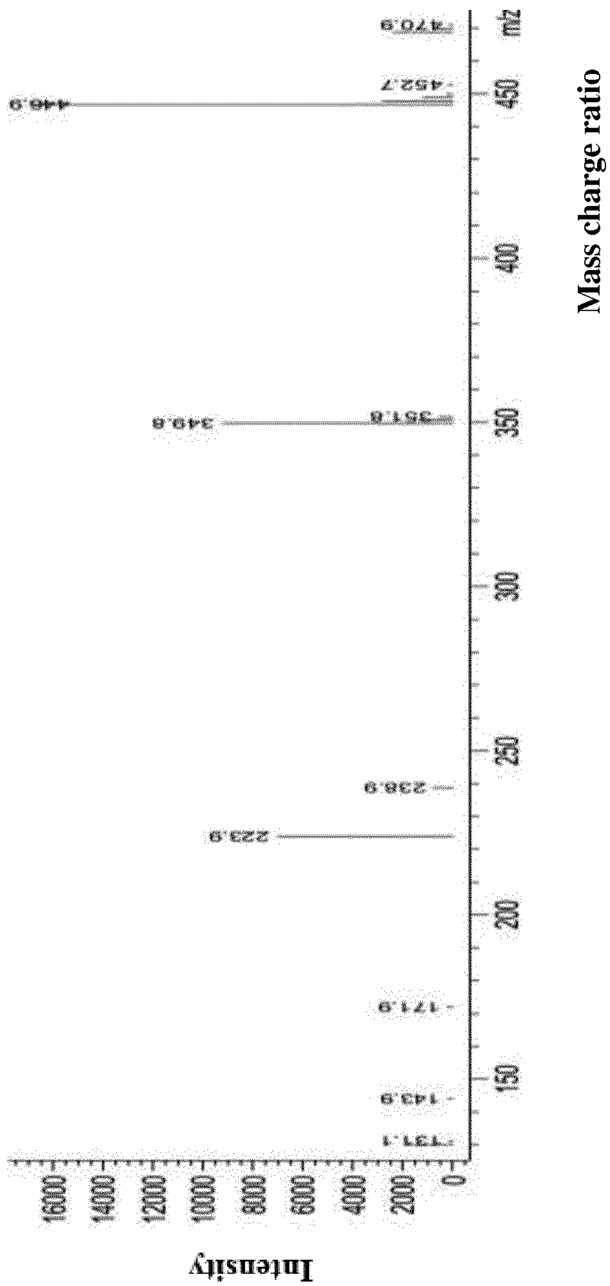
Intensity
Mass charge ratio
m/z
131.1
143.9
171.9
223.9
238.9
349.6
351.6
446.9
452.7
470.9
0
2000
4000
6000
8000
10000
12000
14000
16000
150
200
250
300
350
400
450

**Figure 3**

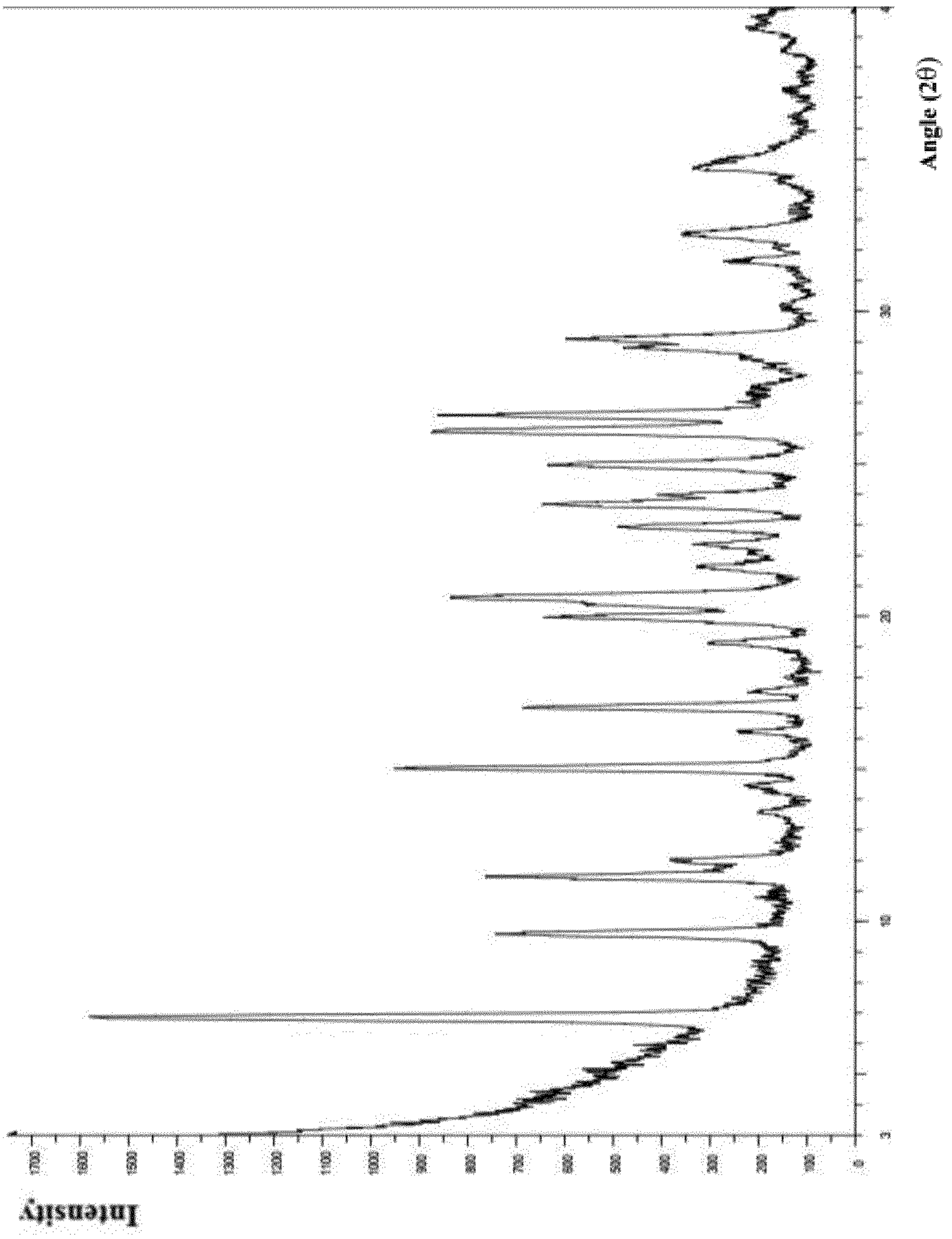

# INTERNATIONAL SEARCH REPORT

International application No. PCT/CN2017/073645

**A. CLASSIFICATION OF SUBJECT MATTER**

A61K 31/506 (2006.01) i; C07D 417/02 (2006.01) i; A61K 9/08 (2006.01) i; A61K 9/10 (2006.01) i; A61K 9/107 (2006.01) i; A61K 9/14 (2006.01) i; A61K 9/19 (2006.01) i; A61K 9/20 (2006.01) i; A61K 9/48 (2006.01) i; A61P 35/00 (2006.01) i; A61P 35/02 (2006.01) i; A61P 19/08 (2006.01) i

According to International Patent Classification (IPC) or to both national classification and IPC

B. FIELDS SEARCHED

Minimum documentation searched (classification system followed by classification symbols)

A61K, C07D, A61P

Documentation searched other than minimum documentation to the extent that such documents are included in the fields searched

Electronic data base consulted during the international search (name of data base and, where practicable, search terms used)

CNABS, CPRSABS, CNMED, MOABS, HKABS, TWABS, DWPI, SIPOABS, JPABS, CNTXT, USTXT, JPTXT, EPTXT, WOTXT, CNKI, CMPD, CPA, PUBMED, STN: Fms-like tyrosine kinase, FLT3, Fetal Liver Kinase-2, FLK2, Stem Cell Kinase-1, STK1, tumour, tumor, cancer, leukemia, leukocythemia, leukaemia, lymphoma, lymphadenoma, myeloma, myelosis, myelofibrosis, thrombocytosis, polyplastocytosis, thrombocythemia, cytopenia, hematocytopenia, myelodysplasia, structure search of formula I

C. DOCUMENTS CONSIDERED TO BE RELEVANT

| Category* | Citation of document, with indication, where appropriate, of the relevant passages | Relevant to claim No. |
|---|---|---|
| X | CN 102924446 A (SHANGHAI JIKAI MEDICAL TECHNOLOGY CO., LTD.) 13 February 2013 (13.02.2013) see the whole document, especially the abstract, claims 1 and 12, and description, paragraphs [0003], [0043], [0073] and [0208]-[0212] | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 12 April 2017 | 24 May 2017 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br>FAN, Chanjuan<br>Telephone No. (86-10) 62411209 |

Form PCT/ISA /210 (second sheet) (July 2009)

# INTERNATIONAL SEARCH REPORT

International application No.
PCT/CN2017/073645

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 8-10
because they relate to subject matter not required to be searched by this Authority, namely:
[1] Claims 8-10 relate to a method of preventing and/or treating a disease or disorder associated with FLT3, belong to a method of treatment of the human or animal body by therapy (PCT Rule 39.1(iv)), but the examiner still searched based on a reasonable expected subject, i.e. the use of a compound of Formula 1 in the preparation of a drug for preventing and/or treating a disease or disorder associated with FLT3.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2017/073645

| Patent Documents referred in the Report | Publication Date | Patent Family | Publication Date |
|---|---|---|---|
| CN 102924446 A | 13 February 2013 | WO 2013020369 A1 | 14 February 2013 |
| | | AU 2012292859 A1 | 27 March 2014 |
| | | JP 6043352 B2 | 14 December 2016 |
| | | US 2014162998 A1 | 12 June 2014 |
| | | CN 102924445 A | 13 February 2013 |
| | | US 2014162999 A1 | 12 June 2014 |
| | | CN 102924445 B | 08 July 2015 |
| | | CA 2844855 A1 | 14 February 2013 |
| | | JP 2014525919 A | 02 October 2014 |
| | | EP 2743269 A4 | 22 April 2015 |
| | | CA 2844852 A1 | 14 February 2013 |
| | | EP 2743271 A4 | 01 April 2015 |
| | | CN 102924444 A | 13 February 2013 |
| | | US 9090594 B2 | 28 July 2015 |
| | | US 8969584 B2 | 03 March 2015 |
| | | CN 102924444 B | 08 July 2015 |
| | | WO 2013020370 A1 | 14 February 2013 |
| | | EP 2743269 A1 | 18 June 2014 |
| | | JP 2014521704 A | 28 August 2014 |
| | | EP 2743271 A1 | 18 June 2014 |
| | | WO 2013020371 A1 | 14 February 2013 |
| | | CN 102924446 B | 26 August 2015 |
| | | AU 2012292858 A1 | 27 March 2014 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201610450691


### Non-patent literature cited in the description


- **ANSARI-LARI.** Ali. FLT3 mutations in myeloid sarcoma. *British Journal of Haematology,* vol. 126 (6), 785-91